# EUROPEAN PATENT APPLICATION

(11) **EP 4 606 788 A1**
(43) Date of publication of application: **27.08.2025**
(21) Application number: 24158777.3
(22) Date of filing: 21.02.2024
(51) Int. Cl.: C07C 209/84, C12P 13/00

(54) **USE OF TWO DIFFERENT SOLVENTS FOR THE EXTRACTION OF 1,6-HEXAMETHYLENEDIAMINE PRODUCED BY MICROBIAL FERMENTATION**

(71) Applicant: Covestro Deutschland AG, 51373 Leverkusen (DE); Genomatica, Inc., San Diego CA 92121 (US)
(72) Inventor: KRAUSE, Jonas, 51373 Leverkusen (DE); ALTUNTEPE, Emrah, 51373 Leverkusen (DE); FAECKE, Thomas, 51373 Leverkusen (DE); GAMEZ, Jose, 51373 Leverkusen (DE); WALTERMANN, Thomas, 51373 Leverkusen (DE)
(74) Representative: Scholz, Volker

(57) **Abstract**

Method for separating 1,6-hexamethylenediamine (HMDA) from an aqueous solution, comprising the steps: a) providing HMDA-containing aqueous solution, wherein said aqueous solution is a fermentation broth, b) extracting the HMDA-containing aqueous solution with a solvent mixture (i) at least one monovalent alcohol having 4 to 7 carbon atoms and (ii) at least one alkane having 5 to 7 carbon atoms, wherein the alkane content of the solvent mixture is in the range from 2 wt% to 50 wt% based on the total weight of all alcohols and alkanes present in the solvent mixture; c) obtaining an HMDA-containing solvent mixture as separatephase, and d) distilling the HMDA-containing solvent mixture and separating the HMDA from the solvent mixture.

## Description

The present invention relates to the extraction of 1,6-hexamethylenediamine from aqueous mixtures originating from a microbial fermentation process.

### Technical Background

Biobased production of 1,6-hexamethylenediamine results in highly diluted aqueous solutions. Different methods for the removal of diamine from water are described in the technical field. An efficient way to remove the diamine from the water and water soluble impurities is the extraction of the diamine using monovalent alcohols followed by distillation. Since diamines, such as 1,6-hexamethylenediamine, are highly polar, their separation with a water-non-miscible organic solvent is not easy and thus the selection of a proper organic solvent is difficult.

It is known that monovalent solvents are selective and sufficient solvents, but due to the alcohols physicochemical properties the removal of water from the diamine is limited and a significant amount of water is transferred to the organic extract phase and needs to be distilled off. This increases energy demand of the process and reduces advantages of the extraction process.

In order to minimize the energy consumption during distillation it is desirable to use a solvent with low polarity. However, diamines, such as 1,6-hexamethylenediamine, themselves are regularly very polar and the partition coefficient decreases with decreasing polarity of the solvent.

EP 2 806 026 A1 relates to the production of 1,5-pentamethylenediamine. Within this process, a separation step from an aqueous solution is described. This separation can be conducted as an extraction process. The extractant can be an organic solvent, preferably an aliphatic alcohol.

US 2017/0369913 A1 describes the extraction of 1,6-hexamethylenediamine with organic solvents from aqueous solutions. Preferably, alcohols are used. However, also alkanes are tested and found to extract only little amounts of 1,6-hexamethylenediamine but also only little amounts of water.. It is not disclosed that a combination of alkanes and alcohols is more advantageous than the use of alcohols or alkanes as single compounds.

### Technical objective

The problem of the present invention was to provide an improved method for extracting 1,6-hexamethylenediamine (HMDA) from aqueous mixtures originating from a microbial fermentation process that offers economic and as far as possible also ecological benefits compared to the state of the art.

### Technical Solution

The technical objective can be solved by a method for separating 1,6-hexamethylenediamine (HMDA) from an aqueous solution, comprising the steps:
a) providing an HMDA-containing aqueous solution, wherein said aqueous solution is a fermentation broth,
b) extracting the HMDA-containing aqueous solution with a solvent mixture
   (i) at least one monovalent alcohol having 4 to 7 carbon atoms and
   (ii) at least one alkane having 5 to 7 carbon atoms, wherein the alkane content of the solvent mixture is in the range from 2 wt.-% to 50 wt.-% based on the total weight of all alcohols and alkanes present in the solvent mixture;
c) obtaining a HMDA-containing solvent mixture, and
d) distilling the HMDA-containing solvent mixture and separating the HMDA from the solvent mixture.

It was found that using a specific mixture of solvents is advantageous for the extraction of HMDA. The first solvent is a monovalent alcohol having 4 to 7 carbon atoms, preferably 1-butanol, 1-pentanol, 1-hexanol, 1-heptanol, cyclopentanol or cyclohexanol. As a second solvent an alkane having 5 to 7 carbon atoms is added, preferably a n-alkane.

Generally, to reduce the energy demand for distillation the amount of water in the organic extract phase can be reduced by modifying the organic solvent properties. To modify the organic solvent properties additives can be introduced, as for example nonpolar solvent like alkanes. However, 1,6-Hexamethylenediamine is highly polar.

Therefore, it was expected that the partitioning into the modified organic solvent phase would be worse. Surprisingly it was found that the modification of the organic extract phase by adding additives leads not only to a reduction of water and energy demand for distillation but also leads to an improved partitioning, if selected carefully. It can be found, that for different systems different optimal combinations and organic solvent / additive ratios exists. Even in cases, where the partition coefficient does not increase, the water content of the mixture of organic solvents and HMDA is decreased leading to a decreased energy consumption during subsequent distillation. This effect has to be balanced with a decreasing HMDA-concentration in the organic phase which increases energy consumption.

### Advantageous technical effect

Firstly, a lower water-content in the extract can be achieved. Secondly, a surprisingly increased partition coefficient can be observed. As set forth above, this technical effect of a unipolar solvent on the extraction of a polar compound was unexpected.

### Detailed description of the invention

In accordance with the invention, the expressions "comprising", "containing" or "including" preferably mean "consisting substantially of' and particularly preferably "consisting of".

Statements concerning the content of organic compounds in the context of the present invention refer, unless stated otherwise, to values determined by gas chromatography. The skilled person is familiar with the quantitative evaluation of gas chromatograms, optionally using an internal standard. The skilled person is likewise familiar with any methods that might perhaps be needed to determine the water content. The methods known in the art can also be used in the context of the present invention. In case of doubt, the water content determined by Karl Fischer titration is decisive. The presence of amines can lead to trailing end points. In such cases, the figure determined by Karl Fischer titration after buffering with anhydrous benzoic acid is decisive. The skilled person is likewise familiar with the method using buffering with benzoic acid. For Karl Fischer titration in general, see Jander, Jahr, Massanalyse, 17th ed., de Gruyter, Berlin (2009), p. 279 to p. 282).

"Distillation" in the present case is understood to mean a thermal separation process used to recover evaporable substances, preferably liquids, from a composition. The separated vapors are subsequently precipitated, usually by condensation. The term encompasses in particular repeated evaporation and condensation using a column (distillation column) with a plurality of separating stages. Processes of this kind in which several distillation steps are arranged in series in a column should strictly speaking be called rectification, but will nevertheless likewise be referred to herein as distillation for the purpose of simplification. The advantage of these processes is the powerful separating effect and the possibility of operating the plant continuously. A "distillation apparatus" is accordingly an apparatus which is suitable for carrying out such a thermal separation process, i.e. for example sieve plate columns, packed columns, packed-bed columns, bubble cap tray columns or even single-stage evaporators such as falling film evaporators, thin-film evaporators, flash evaporators, multi-phase helical-coil evaporators, natural or forced circulation evaporators.

The relevant pressure of a distillation apparatus in the present case is the respective head pressure prevailing in the device, i.e. the pressure at which the vapors are present before they exit the distillation apparatus. This pressure will therefore also be referred to below as head pressure.

The individual process steps will be explained in more detail below.

### Step a)

In the first step a) of the method of the invention, an HMDA-containing aqueous solution which is a fermentation broth is provided.

A "fermentation broth" as referred to in the present application is an aqueous composition which originates from a microbial fermentation process. This microbial fermentation process is, preferably, a process, wherein microorganisms, preferably bacteria, are provided with one or more organic compounds that are converted to HMDA by the metabolic activity of said microorganisms. The term "providing" thus refer to the practice of such a microbial fermentation process. As microbial cells release further products in addition to HMDA into the fermentation broth and a suitable fermentation broth must comprise a range of nutrients such as amino acids or vitamins in order to support the activity of the microorganisms, it is clear that the aqueous solution must comprise organic compounds in addition to HMDA.

Therefore, in a preferred embodiment of the present invention, said aqueous solution additionally comprises at least one compound selected from the group consisting of (i) acetate, (ii) lactate, (iii) glutamate, (iv) ammonium and (v) γ-aminobutyric acid.

The compounds recited above are typical by-products of microbial fermentations. Acetate is a short-chain fatty acid which originates from a multitude of aerobic as well anaerobic metabolic pathways in microorganisms, e.g. butyric acid fermentation or by cleavage of acetyl-coenzyme A. Lactate is a product of glycolysis. Glutamate is a proteinogenic amino acid. Ammonium is found in many fermentation broths as source of nitrogen. It may also be released during amino acid metabolism. γ-aminobutyric acid is produced by a multitude of microorganisms such as certain *Lactobacilli* or yeasts.

Acetate is preferably present in a concentration range between 1 and 4,000 mg/L, more preferably 200 and 3,000 mg/L and most preferably 750 to 2,500 mg/L.

Lactate is preferably present in a concentration range between 1 and 400 mg/L, more preferably 5 and 200 mg/L and most preferably 10 to 100 mg/L.

Glutamate is preferably present in a concentration range between 10 and 1,000 mg/L, more preferably 50 and 600 mg/L and most preferably 75 to 250 mg/L.

Ammonium is preferably present in a concentration range between 1 and 200 mg/L, more preferably 5 and 120 mg/L and most preferably 10 to 80 mg/L.

γ-aminobutyric acid is preferably present in a concentration range between 300 and 4,000 mg/L, more preferably 500 and 3,000 mg/L and most preferably 700 to 2,000 mg/L.

The present application does not relate to the separation of HMDA from an aqueous solution, if the HMDA originates from the depolymerization of a polymer. Such an aqueous solution is not the product of a microbial fermentation process but from the chemical process used for the depolymerization.

Independently of the additional constituents of the aqueous composition comprising HMDA at the end of method step a) the composition comprises 0.1 to 50 wt.-% HMDA, preferably 5 to 20 wt.-% HMDA and most preferably 7 to 22 wt.-% HMDA. The water content of the composition is preferably at least 50 wt.-%, more preferably at least 55 wt.-%. All percentages are based on the total weight of the composition as 100 wt.-%. In addition, the HMDA-containing aqueous solution usually also contains other substances apart from water which have a lower boiling point than HMDA. The content of these substances which have a lower boiling point than the amine is preferably ≤ 10 wt.-%, particularly preferably ≤ 5 wt.-% and most particularly preferably ≤ 3 wt.-%. At the same time, the content in technical processes is usually ≥ 0.01 wt.-%, preferably ≥ 0.3 wt.-%, unless low boilers have already been separated beforehand. Additionally, the HMDA-containing aqueous solution usually also contains other substances which have a higher boiling point than the amine. The proportion of these substances which have a higher boiling point than the amine in the first composition preferably totals ≥0.01 wt.-% and ≤10 wt.-%, particularly preferably ≥0.1 wt.-% and ≤5 wt.-%, and most particularly preferably ≥0.3 wt.-% and ≤3 wt.-%.

Substances with lower boiling points compared to the HMDA that can occur in the HMDA-containing aqueous solution are, apart from water, for example ammonia, alkyl or alkenyl amines, alcohols, ethylene glycol, cyclic amines such as pyrrolidine, piperidine, azepane, imines, cylic imines such as tetrahydropyridine or tetrahydroazepine, or residues of solvents such as hydrocarbons or halogenated hydrocarbons for example. In addition, gases such as nitrogen or carbon dioxide may be present in the solution in dissolved or bound form.

Substances with higher boiling points compared to HMDA that can occur are for example dimers or oligomers of HMDA, which can form while cleaving off ammonia from HMDA.

As the HMDA-containing aqueous solution is produced by biotechnological means, the HMDA -containing aqueous solution may also contain carbohydrates which have not been completely converted in the fermentation process and salts that originate from the fermentation broth and are now still present in the diamine-containing aqueous solution.

The HMDA present in the solution provided in method step a) is preferably present in uncharged form. This is typically the case at a pH of at least 11.5. Therefore, the HMDA-containing aqueous solution in method step a) preferably has a pH of at least 11.5, more preferably 12.0.

### Step b)

In the second step b) of the method of the invention, the HMDA -containing aqueous solution is extracted with a solvent mixture comprising (i) at least one monovalent alcohol having 4 to 7 carbon atoms; and (ii) at least one alkane having 5 to 7 carbon atoms, wherein the alkane content of the solvent mixture is in the range from 2 wt.-% to 50 wt.-% based on the total weight of all alcohols and alkanes present in the solvent mixture.

Preferably, the at least one monovalent alcohol having 4 to 7 carbon atoms is selected from the group consisting of 1-butanol, 1-pentanol, 1-hexanol, 1-heptanol, cyclopentanol, cyclohexanol and mixtures thereof. More preferably, the at least one monovalent alcohol having 4 to 7 carbon atoms is selected from the group consisting of 1-butanol, 1-pentanol, 1-hexanol and mixtures thereof. Most preferably, the at least one monovalent alcohol having 4 to 7 carbon atoms is 1-hexanol.

The at least one alkane having 5 to 7 carbon atoms may be linear, branched or cyclic. More preferably, the at least one alkane having 5 to 7 carbon atoms may be linear or cyclic. Most preferably, the at least one alkane having 5 to 7 carbon atoms may be linear.

Preferably, the at least one alkane having 5 to 7 carbon atoms is selected from n-pentane, n-hexane, n-heptane and mixtures thereof.

Preferably, the alkane content of the solvent mixture is in the range from 2 wt.-% to 40 wt.-%, more preferably 2 wt.-% to 30 wt.-%, most preferably 2 wt.-% to 20 wt.-% based on the total weight of all alcohols and alkanes present in the solvent mixture.

### Step c)

In the third step c) of the method of the invention, an HMDA-containing solvent mixture is obtained as a separate phase. The measures in order to obtain the HMDA-containing solvent mixture as a separate phase are not particularly limited. In general, the HMDA-containing solvent mixture can be separated from the aqueous phase after step b) as soon as two distinct phases are formed. The process can be performed in a mixer-settler, extraction column or centrifugal separator with 1 to 20 stages, preferably 3 to 15 stages, most preferably 5 to 10 stages.

### Step d)

In the fourth step d) of the method of the invention, the HMDA-containing solvent mixture is distilled and the HMDA is separated from the solvent mixture.

A distillation device used in step d) is not limited, but any distillation device known in the art for such separation steps may be used. The distillation device may be a single distillation column. The single distillation column may have 5 to 100, 10 to 60 or 20 to 50 theoretical stages. In case, 1-hexanol is the monovalent alcohol, 20 or more theoretical stages, e.g., 20 to 50 theoretical stages, may be used. The distillation pressure may be from 100-1,000 mbar, 150-800 mbar, 150-700 mbar, 200-600 mbar, 200-500 mbar or 200-400 mbar. It is, thus, lower than the atmospheric pressure of 1,013 mbar.

Any combinations of embodiments, especially preferred, more preferred, most preferred ranges and/or embodiments are suitable and selectable and are particularly preferred.

### Brief description of the drawings

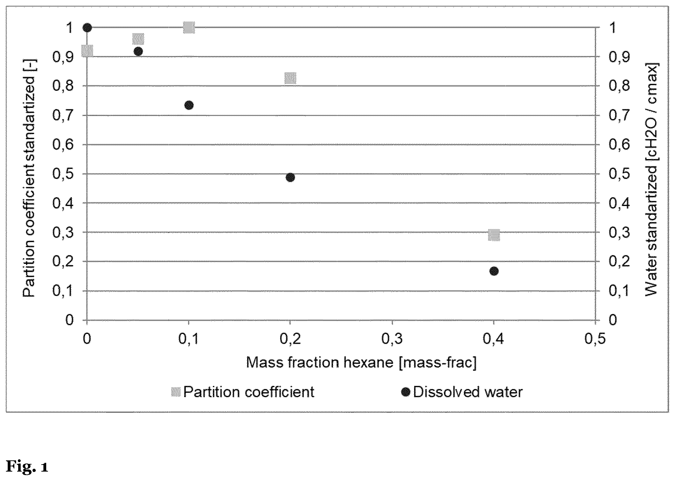

Fig. 1 is a graph illustrating the dependency of the partition coefficient and the water content of the solvent mixture on the proportion of butanol and hexane as analyzed in example 1;

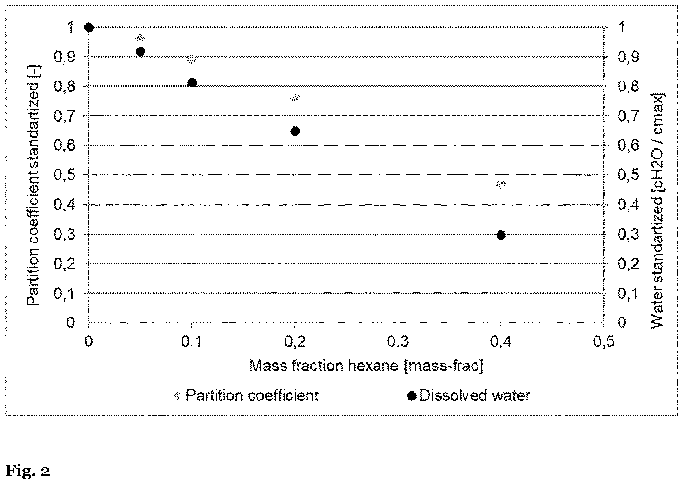

Fig. 2 is a graph illustrating the dependency of the partition coefficient and the water content of the solvent mixture on the proportion of hexanol and hexane as analyzed in example 2.

The present invention will be described in more detail in the following examples. However, these examples are for illustrative purposes only.

### Examples

### Example 1: Extraction of HMDA using 1-Butanol

The aqueous phase was synthetically created by dissolving 10 wt.-% of HMDA in 1 molar aqueous NaOH. The organic phase was created by mixing various concentrations of n-hexane and 1-butanol. The organic phase was saturated with water before used for extraction as the organic phase. 100 g of the aqueous solution was mixed with 35 g of the water saturated organic phase and the temperature was adjusted to 50 °C. Mixing was then continued for at least 10 minutes to reach equilibrium. Afterwards the phases were settled by gravity until both phases were completely separated. The concentration of HMDA was measured via HPLC-MS in both phases. For evaluation the partition coefficient was calculated via the concentration in the organic phase over the concentration of HMDA in the aqueous phase and standardized to the highest value measured. In addition, the dissolved water was measured via Karl-Fischer titration in the organic alcohol/alkane mixture and standardized to the highest value measured.

A mass fraction of hexane in the organic phase in the range of 5 to 10 wt.-% increases the partition coefficient. Moreover, the water content of the organic phase decreased with increasing concentrations of hexane.

The aqueous phase was synthetically created by dissolving 10 wt.-% of HMDA in 1 molar aqueous NaOH. The organic phase was created by mixing various concentrations of n-hexane and 1-hexanol. The organic phase was saturated with water before used for extraction as the organic phase. 100 g of the aqueous solution was mixed with 35 g of the water saturated organic phase and the temperature was adjusted to 50 °C. Mixing was then continued for at least 10 minutes to reach equilibrium. Afterwards the phases were settled by gravity until both phases were completely separated. The concentration of HMDA was measured via HPLC-MS in both phases. For evaluation the partition coefficient was calculated via the concentration in the organic phase over the concentration of HMDA in the aqueous phase and standardized to the highest value measured. In addition, the dissolved water was measured via Karl-Fischer titration in the organic alcohol/alkane mixture and standardized to the highest value measured. With 1-hexanol the addition of hexane did not increase the partition coefficient. Nevertheless, the decreased water content of the organic phase concomitantly decreases the energy requirements during separation of the HMDA from the solvents during distillation as long as it is not too high. Generally, a mass fraction of hexane of 40 wt.-% is the limit, where the decreased energy consumption due to decreased water content of the organic phase is offset by the increased energy consumption due to the need to remove too much organic solvent.

## Claims

1. Method for separating 1,6-hexamethylenediamine (HMDA) from an aqueous solution, comprising the steps:
a) providing an HMDA-containing aqueous solution, wherein said aqueous solution is a fermentation broth,
b) extracting the HMDA-containing aqueous solution with a solvent mixture
(i) at least one monovalent alcohol having 4 to 7 carbon atoms and
(ii) at least one alkane having 5 to 7 carbon atoms, wherein the alkane content of the solvent mixture is in the range from 2 wt.-% to 50 wt.-% based on the total weight of all alcohols and alkanes present in the solvent mixture;
c) obtaining an HMDA-containing solvent mixture as a separate phase, and
d) distilling the HMDA-containing solvent mixture and separating the HMDA from the solvent mixture.

2. The method of claim 1, wherein the aqueous solution comprises at least one compound selected from the group consisting of (i) acetate, (ii) lactate, (iii) glutamate, (iv) ammonium and (v) γ-aminobutyric acid.

3. The method of claim 2, wherein the concentration range of acetate is between 1 and 4,000 mg/L, the concentration range of lactate is between 1 and 400 mg/L, the concentration range of glutamate is between 10 and 1,000 mg/L, the concentration range of ammonium is between 1 and 200 mg/L, or the concentration range of γ-aminobutyric acid is between 300 and 4,000 mg/L

4. The method according to any of the preceding claims, wherein the at least one monovalent alcohol having 4 to 7 carbon atoms is selected from the group consisting of 1-butanol, 1-pentanol, 1-hexanol, 1-heptanol, cyclopentanol, cyclohexanol and mixtures thereof.

5. The method according to any of the preceding claims, wherein the at least one alkane having 5 to 7 carbon atoms is a linear alkane.

6. The method according to any of the preceding claims, wherein the alkane content of the solvent mixture is in the range from 2 wt.-% to 30 wt.-% based on the total weight of all alcohols and alkanes present in the solvent mixture.

7. The method according to any of the preceding claims, wherein the alkane content of the solvent mixture is in the range from 2 wt.-% to 20 wt.-% based on the total weight of all alcohols and alkanes present in the solvent mixture.
